# EUROPEAN PATENT SPECIFICATION

(11) **EP 3 555 295 B1**
(45) Date of publication and mention of the grant of the patent: **20.01.2021**
(21) Application number: 17823300.3
(22) Date of filing: 12.12.2017
(51) Int. Cl.: C12N 15/62, C12N 9/10, C12N 9/88, C12P 7/06

(54) **BIFUNCTIONAL PHOSPHOKETOLASE-PHOSPHOTRANSACETYLASE FUSION POLYPEPTIDES**
BIFUNKTIONALE PHOSPHOKETOLASE-PHOSPHOTRANSACETYLASE-FUSIONSPOLYPEPTIDE
POLYPEPTIDES DE FUSION PHOSPHOCÉTOLASE-PHOSPHOTRANSACÉTYLASE BIFONCTIONNELS

(30) Priority: 16.12.2016 US 201662435212 P; 16.06.2017 US 201762520604 P; 11.09.2017 US 201762556788 P
(43) Date of publication of application: 23.10.2019
(73) Proprietor: Danisco US Inc., Palo Alto, California 94304 (US)
(72) Inventor: ZHU, Quinn Qun, Palo Alto, California 94304 (US); TEUNISSEN, Paula Johanna, Palo Alto, California 94304 (US); KOZAK, Barbara Urszula, Palo Alto, California 94304 (US); HOLLANDS, Kerry, Palo Alto, California 94304 (US)
(74) Representative: Mewburn Ellis LLP
(86) International application number: PCT/US2017/065680
(87) International publication number: WO 2018/111792

(56) References cited:
- WO-A1-2015/148272
- WO-A2-00/06747
- WO-A2-2015/127305

## Description

### TECHNICAL FIELD

The present compositions and methods relate to bifunctional phosphoketolase-phosphotransacetylase fusion polypeptides and the use thereof in starch hydrolysis processes for alcohol production.

### BACKGROUND

Yeast-based ethanol production is based on the conversion of sugars into ethanol. The current annual fuel ethanol production by this method is about 90 billion liters worldwide. It is estimated that about 70% of the cost of ethanol production is the feedstock. Since the ethanol production volume is so large, even small yield improvements have massive economic impact for the industry. The conversion of one mole of glucose into two moles of ethanol and two moles of carbon dioxide is redox-neutral, with the maximum theoretical yield being about 51%. The current industrial yield is about 45%; therefore, there are opportunities to increase ethanol production.

Carbon dioxide, glycerol and yeast biomass are the major by-products of ethanol fermentation. During yeast growth and fermentation, a surplus of NADH is generated, which is used to produce glycerol for the purposes of redox balance and osmotic protection. Glycerol is considered a low value product and several approaches have been taken to reduce glycerol production. However, reducing glycerol synthesis can result in the increase of other metabolic by-products, such as acetate. Acetate is not a desirable by-product, as it adversely affects the alcohol production rate, titer and yield of yeast fermentation.

The need exists to modify yeast metabolic pathways to maximize ethanol production, while not increasing the production of undesirable pathway by-products such as acetate.

### SUMMARY

The present compositions and methods relate to bifunctional phosphoketolase-phosphotransacetylase fusion polypeptides and the use thereof in starch hydrolysis processes for ethanol production. Aspects and embodiments will be apparent from the description, including the accompanying Figures.

### BRIEF DESCRIPTION OF THE DRAWINGS

Figure 1 is a diagram of the engineered phosphoketolase pathway for producing ethanol and acetate from sugars.
Figure 2 is the amino acid sequence of phosphoketolase and phosphotransacetylase fusion protein 1 (GvPKL-L1-LpPTA; SEQ ID NO: 5). In the fusion protein, GvPKL is at N-terminus, LpPTA is at C-terminus, and linker 1 (L1) is located between, indicated in bold italic.
Figure 3 is a map of plasmid pZK41W-GLAF12. pZK41W-GLAF22 is similar but has a different linker.
Figure 4 is the amino acid sequence of phosphoketolase and phosphotransacetylase fusion protein 2 (GvPKL-L2-LpPTA; SEQ ID NO: 6). In the fusion protein, GvPKL is at N-terminus, LpPTA is at C-terminus, and linker 2 (L2) is located between, indicated in bold italic.
Figure 5 is a map of plasmid pZK41W-(H3C19).
Figure 6 is a map of plasmid pTOPO II-Blunt ura3-loxP-KanMX-loxP-ura3.
Figure 7 is a map of the 2,018-bp *Eco*RI fragment from plasmid pTOPO II-Blunt ura3-loxP-KanMX-loxP-ura3.
Figure 8 is a map of plasmid pGAL-Cre-316.
Figure 9 is a map of the *Swa*I fragment from plasmid pZK41W-GLAF12
Figure 10 is a map of the *Swa*I fragment from plasmid pZK41W-GLAF22
Figure 11 is a map of the *Swa*I fragment from plasmid pZK41W-(H3C19)
Figures 12A and 12B are graphs showing comparisons of the performance of strains POL-SC-00675 (●), POL-SC-0692 (○), POL-SC-0696 (Δ) and POL-SC-00699 (▲) in shake flask assays using liquefact as a substrate at 32°C and 36°C. Strains expressing fused PKL-PTA polypeptide are represented by solid symbols and strains expressing two separate polypeptides of PKA and PTA are represented by open symbols.
Figure 13 is a graph showing a comparison of the performance of strains POL-SC-0696 (Δ) and POL-SC-00699 (▲) in shake flask assays using non-liquefied corn flour as a substrate at 32°C and 36°C. Strains expressing fused PKL-PTA polypeptide are represented by solid symbols and strains expressing two separate polypeptides of PKA and PTA are represented by open symbols.

### DETAILED DESCRIPTION

### I. Definitions

Prior to describing the present yeast strains and methods in detail, the following terms are defined for clarity. Terms not defined should be accorded their ordinary meanings as used in the relevant art.

As used herein, "alcohol" refer to an organic compound in which a hydroxyl functional group (-OH) is bound to a saturated carbon atom.

As used herein, "yeast cells" yeast strains, or simply "yeast" refer to organisms from the phyla Ascomycota and Basidiomycota. An exemplary yeast is budding yeast from the order Saccharomycetales. A particular example of yeast is *Saccharomyces* spp., including but not limited to *S. cerevisiae.* Yeast include organisms used for the production of fuel alcohol as well as organisms used for the production of potable alcohol, including specialty and proprietary yeast strains used to make distinctive-tasting beers, wines, and other fermented beverages.

As used herein, the phrase "engineered yeast cells," "variant yeast cells," "modified yeast cells," or similar phrases, refer to yeast that include genetic modifications and characteristics described herein. Variant/modified yeast do not include naturally occurring yeast.

As used herein, the terms "polypeptide" and "protein" (and their respective plural forms) are used interchangeably to refer to polymers of any length comprising amino acid residues linked by peptide bonds. The conventional one-letter or three-letter codes for amino acid residues are used herein and all sequence are presented from an N-terminal to C-terminal direction. The polymer can comprise modified amino acids, and it can be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

As used herein, functionally and/or structurally similar proteins are considered to be "related proteins." Such proteins can be derived from organisms of different genera and/or species, or different classes of organisms (e.g., bacteria and fungi), or artificially designed. Related proteins also encompass homologs determined by primary sequence analysis, determined by secondary or tertiary structure analysis, or determined by immunological cross-reactivity.

As used herein, the term "homologous protein" refers to a protein that has similar activity and/or structure to a reference protein. It is not intended that homologs necessarily be evolutionarily related. Thus, it is intended that the term encompass the same, similar, or corresponding enzyme(s) (*i.e.,* in terms of structure and function) obtained from different organisms. In some embodiments, it is desirable to identify a homolog that has a quaternary, tertiary and/or primary structure similar to the reference protein. In some embodiments, homologous proteins induce similar immunological response(s) as a reference protein. In some embodiments, homologous proteins are engineered to produce enzymes with desired activity (ies).

The degree of homology between sequences can be determined using any suitable method known in the art (see, *e.g.,* Smith and Waterman (1981) Adv. Appl. Math. 2:482; Needleman and Wunsch (1970) J. Mol. Biol., 48:443; Pearson and Lipman (1988) Proc. Natl. Acad. Sci. USA 85:2444; programs such as GAP, BESTFIT, FASTA, and TFASTA in the Wisconsin Genetics Software Package (Genetics Computer Group, Madison, WI); and Devereux et al. (1984) Nucleic Acids Res. 12:387-95).

For example, PILEUP is a useful program to determine sequence homology levels. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle, (Feng and Doolittle (1987) J. Mol. Evol. 35:351-60). The method is similar to that described by Higgins and Sharp ((1989) CABIOS 5:151-53). Useful PILEUP parameters including a default gap weight of 3.00, a default gap length weight of 0.10, and weighted end gaps. Another example of a useful algorithm is the BLAST algorithm, described by Altschul et al. ((1990) J. Mol. Biol. 215:403-10) and Karlin et al. ((1993) Proc. Natl. Acad. Sci. USA 90:5873-87). One particularly useful BLAST program is the WU-BLAST-2 program (see, *e.g.,* Altschul et al. (1996) Meth. Enzymol. 266:460-80). Parameters "W," "T," and "X" determine the sensitivity and speed of the alignment. The BLAST program uses as defaults a word-length (W) of 11, the BLOSUM62 scoring matrix (see, *e.g.,* Henikoff and Henikoff (1989) Proc. Natl. Acad. Sci. USA 89:10915) alignments (B) of 50, expectation (E) of 10, M'5, N'-4, and a comparison of both strands.

As used herein, the phrases "substantially similar" and "substantially identical," in the context of at least two nucleic acids or polypeptides, typically means that a polynucleotide or polypeptide comprises a sequence that has at least about 70% identity, at least about 75% identity, at least about 80% identity, at least about 85% identity, at least about 90% identity, at least about 91% identity, at least about 92% identity, at least about 93% identity, at least about 94% identity, at least about 95% identity, at least about 96% identity, at least about 97% identity, at least about 98% identity, or even at least about 99% identity, or more, compared to the reference (*i.e.,* wild-type) sequence. Percent sequence identity is calculated using CLUSTAL W algorithm with default parameters. *See* Thompson et al. (1994) Nucleic Acids Res. 22:4673-4680. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF |

Another indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another indication that two nucleic acid sequences are substantially identical is that the two molecules hybridize to each other under stringent conditions (*e.g.*, within a range of medium to high stringency).

As used herein, the term "gene" is synonymous with the term "allele" in referring to a nucleic acid that encodes and directs the expression of a protein or RNA. Vegetative forms of filamentous fungi are generally haploid, therefore a single copy of a specified gene (*i.e.*, a single allele) is sufficient to confer a specified phenotype.

As used herein, the term "expressing a polypeptide" and similar terms refers to the cellular process of producing a polypeptide using the translation machinery (*e.g*., ribosomes) of the cell.

As used herein, the terms "fused" and "fusion" with respect to two polypeptides refer to a physical linkage causing the polypeptide to become a single molecule.

As used herein, the terms "wild-type" and "native" are used interchangeably and refer to genes, proteins or strains found in nature.

As used herein, the term "protein of interest" refers to a polypeptide that is desired to be expressed in modified yeast. Such a protein can be an enzyme, a substrate-binding protein, a surface-active protein, a structural protein, a selectable marker, or the like, and can be expressed at high levels. The protein of interest is encoded by a modified endogenous gene or a heterologous gene (*i.e.*, gene of interest") relative to the parental strain. The protein of interest can be expressed intracellularly or as a secreted protein.

As used herein, "deletion of a gene," refers to its removal from the genome of a host cell. Where a gene includes control elements (*e.g*., enhancer elements) that are not located immediately adjacent to the coding sequence of a gene, deletion of a gene refers to the deletion of the coding sequence, and optionally adjacent enhancer elements, including but not limited to, for example, promoter and/or terminator sequences, but does not require the deletion of non-adjacent control elements.

As used herein, "disruption of a gene" refers broadly to any genetic or chemical manipulation, *i.e.,* mutation, that substantially prevents a cell from producing a function gene product, *e.g*., a protein, in a host cell. Exemplary methods of disruption include complete or partial deletion of any portion of a gene, including a polypeptide-coding sequence, a promoter, an enhancer, or another regulatory element, or mutagenesis of the same, where mutagenesis encompasses substitutions, insertions, deletions, inversions, and combinations and variations, thereof, any of which mutations substantially prevent the production of a function gene product. A gene can also be disrupted using RNAi, antisense, or any other method that abolishes gene expression. A gene can be disrupted by deletion or genetic manipulation of non-adjacent control elements.

As used herein, the terms "genetic manipulation" and "genetic alteration" are used interchangeably and refer to the alteration/change of a nucleic acid sequence. The alteration can include but is not limited to a substitution, deletion, insertion or chemical modification of at least one nucleic acid in the nucleic acid sequence.

As used herein, a "functional polypeptide/protein" is a protein that possesses an activity, such as an enzymatic activity, a binding activity, a surface-active property, or the like, and which has not been mutagenized, truncated, or otherwise modified to abolish or reduce that activity. Functional polypeptides can be thermostable or thermolabile, as specified.

As used herein, "a functional gene" is a gene capable of being used by cellular components to produce an active gene product, typically a protein. Functional genes are the antithesis of disrupted genes, which are modified such that they cannot be used by cellular components to produce an active gene product, or have a reduced ability to be used by cellular components to produce an active gene product.

As used herein, yeast cells have been "modified to prevent the production of a specified protein" if they have been genetically or chemically altered to prevent the production of a functional protein/polypeptide that exhibits an activity characteristic of the wild-type protein. Such modifications include, but are not limited to, deletion or disruption of the gene encoding the protein (as described, herein), modification of the gene such that the encoded polypeptide lacks the aforementioned activity, modification of the gene to affect post-translational processing or stability, and combinations, thereof.

As used herein, "attenuation of a pathway or "attenuation of the flux through a pathway" *i.e.,* a biochemical pathway, refers broadly to any genetic or chemical manipulation that reduces or completely stops the flux of biochemical substrates or intermediates through a metabolic pathway. Attenuation of a pathway may be achieved by a variety of well-known methods. Such methods include but are not limited to: complete or partial deletion of one or more genes, replacing wild-type alleles of these genes with mutant forms encoding enzymes with reduced catalytic activity or increased Km values, modifying the promoters or other regulatory elements that control the expression of one or more genes, engineering the enzymes or the mRNA encoding these enzymes for a decreased stability, misdirecting enzymes to cellular compartments where they are less likely to interact with substrate and intermediates, the use of interfering RNA, and the like.

As used herein, "aerobic fermentation" refers to growth in the presence of oxygen.

As used herein, "anaerobic fermentation" refers to growth in the absence of oxygen.

As used herein, the singular articles "a," "an," and "the" encompass the plural referents unless the context clearly dictates otherwise. The following abbreviations/acronyms have the following meanings unless otherwise specified:
- EC: enzyme commission
- PKL: phosphoketolase
- PTA: phosphotransacetylase
- XFP: xylulose 5-phosphate/fructose 6-phosphate phosphoketolase
- AADH: acetaldehyde dehydrogenases
- ADH: alcohol dehydrogenase
- EtOH: ethanol
- AA: α-amylase
- GA: glucoamylase
- °C: degrees Centigrade
- bp: base pairs
- DNA: deoxyribonucleic acid
- DP: degree of polymerization
- ds or DS: dry solids
- g or gm: gram
- g/L: grams per liter
- GAU/g ds: glucoamylase units per gram dry solids
- H₂O: water
- HPLC: high performance liquid chromatography
- hr or h: hour
- kg: kilogram
- M: molar
- mg: milligram
- mL or ml: milliliter
- ml/min: milliliter per minute
- mM: millimolar
- N: normal
- nm: nanometer
- OD: optical density
- PCR: polymerase chain reaction
- ppm: parts per million
- SSCU/g ds: fungal alpha-amylase units per gram dry solids
- Δ: relating to a deletion
- µg: microgram
- µL and µl: microliter
- µM: micromolar
- SSF: simultaneous saccharification and fementation
- MTP: microtiter plate

### II. Engineered yeast cells producing a bifunctional PKL-PTA fusion polypeptide

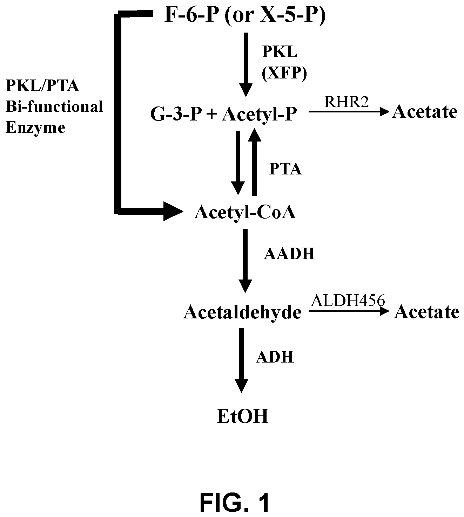

Engineered yeast cells having a heterologous phosphoketolase pathway have been previously described (*e.g*., WO2015148272). These cells express heterologous phosphoketolase (PKL; EC 4.1.2.9) and phosphotransacetylase (PTA; EC 2.3.1.8), optionally with other enzymes, to channel carbon flux away from the glycerol pathway and toward the synthesis of acetyl-coA, which is then converted to ethanol (Figure 1). These cells are capable of increased ethanol production in a fermentation process when compared to otherwise-identical parent yeast cells. Unfortunately, such modified also produce increased acetate, which adversely affect cell growth and represents a "waste" of carbon.

It has now been discovered that ethanol yield can be increased and acetate production reduced by engineering yeast cells to produce a bifunctional PKL-PTA fusion polypeptide, which includes active portions of both enzymes. Over-expression of such bifunctional fusion polypeptides increases ethanol yield while reducing acetate production up to 37% compared to the over-expression of the separate enzymes.

With continuing reference to Figure 1, and without being limited to a theory, it is believed that the expression of separate heterologous PKL and PTA enzymes in a yeast cell allows the production of the intermediate glyceraldehyde-3-phosphate (G-3-P) and acetyl-phosphate (Acetyl-P), the latter being converted to unwanted acetate by an endogenous promiscuous glycerol-3-phosphatase with acetyl-phosphatase activity (GPP1/RHR2). However, by expressing a bifunctional PKL-PTA fusion polypeptide, acetyl-phosphate is rapidly converted to acetyl-CoA, reducing the accumulation of acetyl-phosphate, thereby reducing acetate production. Accordingly, the fusion protein provides a mechanism for the efficient conversion of fructose-6-P (F-6-P) and/or xylulose-5-P (X-5-P) to acetyl-CoA.

As evidenced by the experimental data described, herein, PKL-PTA fusion polypeptides performed according to the foregoing hypothesis. These fusion polypeptides included different linkers between the PKL and PTA polypeptide. It is fully expected that using different PKL and PTA polypeptides will produce similar results. It is also expected that the orientation of the PKL and PTA polypeptides within the fusion polypeptide is not critical, and that either the PKL or the polypeptide can be at the N-terminus or C-terminus of the fusion polypeptide. Numerous PKL and PTA enzymes have been described in the art and should be considered alternatives to the exemplified enzymes.

It is further expected that the PKL-PTA fusion polypeptide can include additional functionalities and structural features within the linker region, including but not limited to, fluorescent proteins, additional enzymes, antibody tags, antibiotic resistance markers, and the like, which may be at the N-terminus and/or C-terminus of the PKL-PTA fusion polypeptide, may separate the PKL-PTA fusion polypeptide, or may be contained within a linker region of a PKL-PTA fusion polypeptide.

### III. Exemplary PKL and PTA fusion polypeptides

In some particular embodiments, the PKL-PTA fusion polypeptide is one of the exemplified bifunctional PKL-PTA fusion polypeptide. The fusion polypeptides are relatively simple in design in that they include full-length PKL from *Gardnerella vaginalis* (*i.e.*, GvPKL) and full-length PTA from *Lactobacillus plantarum* (*i.e.*, LpPTA) connected via one of two different linkers (*i.e.,* L1 or L2).

The amino acid sequence of *G*. *vaginalis* PKL is shown, below (SEQ ID NO: 1):

MTSPVIGTPWKKLNAPVSEAAIEGVDKYWRVANYLSIGQIYLRSNPLMKEPFTREDVKHRLV
GHWGTTPGLNFLIGHINRFIAEHQQNTVIIMGPGHGGPAGTAQSYLDGTYTEYYPKITKDEA
GLQKFFRQFSYPGGIPSHFAPETPGSIHEGGELGYALSHAYGAVMNNPSLFVPAIVGDGEAE
TGPLATGWQSNKLVNPRTDGIVLPILHLNGYKIANPTILSRISDEELHEFFHGMGYEPYEFV
AGFDDEDHMSIHRRFADMFETIFDEICDIKAEAQTNDVTRPFYPMIIFRTPKGWTCPKFIDG
KKTEGSWRAHQVPLASARDTEAHFEVLKNWLKSYKPEELFNEDGSIKEDVLSFMPQGELRIG
QNPNANGGRIREDLKLPNLDDYEVKEVKEFGHGWGQLEATRRLGVYTRDVIKNNPDSFRIFG
PDETASNRLQAAYEVTNKQWDAGYLSELVDEHMAVTGQVTEQLSEHQMEGFLEAYLLTGRHG
IWSSYESFVHVIDSMLNQHAKWLEATVREIPWRKPISSMNLLVSSHVWRQDHNGFSHQDPGV

TSVLLNKTFNNDHVIGIYFPVDSNMLLAVGEKVYKSTNMINAIFAGKQPAATWLTLDEAREE
LEKGAAEWKWASNAKNNDEVQVVLAGIGDVPQQELMAAADKLNKLGVKFKVVNIVDLLKLQS
AKENNEALTDEEFTELFTADKPVLLAYHSYAHDVRGLIFDRPNHDNFNVHGYKEQGSTTTPY
DMVRVNDMDRYELTAEALRMVDADKYADEIKKLEDFRLEAFQFAVDKGYDHPDYTDWVWPGV
KTDKPGAVTATAATAGDNE

The amino acid sequence of *L. plantarum* PTA is shown, below (SEQ ID NO: 2):

MDLFESLAQKITGKDQTIVFPEGTEPRIVGAAARLAADGLVKPIVLGATDKVQAVANDLNAD
LTGVQVLDPATYPAEDKQAMLDALVERRKGKNTPEQAAKMLEDENYFGTMLVYMGKADGMVS
GAIHPTGDTVRPALQIIKTKPGSHRISGAFIMQKGEERYVFADCAINIDPDADTLAEIATQS
AATAKVFDIDPKVAMLSFSTKGSAKGEMVTKVQEATAKAQAAEPELAIDGELQFDAAFVEKV
GLQKAPGSKVAGHANVFVFPELQSGNIGYKIAQRFGHFEAVGPVLQGLNKPVSDLSRGCSEE
DVYKVAIITAAQGLA

The amino acid sequence of Linker 1 (L1) is shown, below (SEQ ID NO: 3): GAGPARPAGLPPATYYDSLAVTS

The amino acid sequence of Linker 2 (L2) is shown, below (SEQ ID NO: 4): AGGGGTS

The amino acid sequence of GvPKL-L1-LpPTA is shown, below (SEQ ID NO: 5), with the linker in bold and italic:

MTSPVIGTPWKKLNAPVSEAAIEGVDKYWRVANYLSIGQIYLRSNPLMKEPFTREDVKHRLV
GHWGTTPGLNFLIGHINRFIAEHQQNTVIIMGPGHGGPAGTAQSYLDGTYTEYYPKITKDEA
GLQKFFRQFSYPGGIPSHFAPETPGSIHEGGELGYALSHAYGAVMNNPSLFVPAIVGDGEAE
TGPLATGWQSNKLVNPRTDGIVLPILHLNGYKIANPTILSRISDEELHEFFHGMGYEPYEFV
AGFDDEDHMSIHRRFADMFETIFDEICDIKAEAQTNDVTRPFYPMIIFRTPKGWTCPKFIDG
KKTEGSWRAHQVPLASARDTEAHFEVLKNWLKSYKPEELFNEDGSIKEDVLSFMPQGELRIG
QNPNANGGRIREDLKLPNLDDYEVKEVKEFGHGWGQLEATRRLGVYTRDVIKNNPDSFRIFG
PDETASNRLQAAYEVTNKQWDAGYLSELVDEHMAVTGQVTEQLSEHQMEGFLEAYLLTGRHG
IWSSYESFVHVIDSMLNQHAKWLEATVREIPWRKPISSMNLLVSSHVWRQDHNGFSHQDPGV
TSVLLNKTFNNDHVIGIYFPVDSNMLLAVGEKVYKSTNMINAIFAGKQPAATWLTLDEAREE
LEKGAAEWKWASNAKNNDEVQVVLAGIGDVPQQELMAAADKLNKLGVKFKVVNIVDLLKLQS
AKENNEALTDEEFTELFTADKPVLLAYHSYAHDVRGLIFDRPNHDNFNVHGYKEQGSTTTPY
DMVRVNDMDRYELTAEALRMVDADKYADEIKKLEDFRLEAFQFAVDKGYDHPDYTDWVWPGV
KTDKPGAVTATAATAGDNEGAGPARPAGLPPATYYDSLAVTSMDLFESLAQKITGKDQTIVF

PEGTEPRIVGAAARLAADGLVKPIVLGATDKVQAVANDLNADLTGVQVLDPATYPAEDKQAM
LDALVERRKGKNTPEQAAKMLEDENYFGTMLVYMGKADGMVSGAIHPTGDTVRPALQIIKTK
PGSHRISGAFIMQKGEERYVFADCAINIDPDADTLAEIATQSAATAKVFDIDPKVAMLSFST
KGSAKGEMVTKVQEATAKAQAAEPELAIDGELQFDAAFVEKVGLQKAPGSKVAGHANVFVFP
ELQSGNIGYKIAQRFGHFEAVGPVLQGLNKPVSDLSRGCSEEDVYKVAIITAAQGLA

The amino acid sequence of GvPKL-L2-LpPTA is shown, below (SEQ ID NO: 6) with the linker in bold and italic:

MTSPVIGTPWKKLNAPVSEAAIEGVDKYWRVANYLSIGQIYLRSNPLMKEPFTREDVKHRLV
GHWGTTPGLNFLIGHINRFIAEHQQNTVIIMGPGHGGPAGTAQSYLDGTYTEYYPKITKDEA
GLQKFFRQFSYPGGIPSHFAPETPGSIHEGGELGYALSHAYGAVMNNPSLFVPAIVGDGEAE
TGPLATGWQSNKLVNPRTDGIVLPILHLNGYKIANPTILSRISDEELHEFFHGMGYEPYEFV
AGFDDEDHMSIHRRFADMFETIFDEICDIKAEAQTNDVTRPFYPMIIFRTPKGWTCPKFIDG
KKTEGSWRAHQVPLASARDTEAHFEVLKNWLKSYKPEELFNEDGSIKEDVLSFMPQGELRIG
QNPNANGGRIREDLKLPNLDDYEVKEVKEFGHGWGQLEATRRLGVYTRDVIKNNPDSFRIFG
PDETASNRLQAAYEVTNKQWDAGYLSELVDEHMAVTGQVTEQLSEHQMEGFLEAYLLTGRHG
IWSSYESFVHVIDSMLNQHAKWLEATVREIPWRKPISSMNLLVSSHVWRQDHNGFSHQDPGV
TSVLLNKTFNNDHVIGIYFPVDSNMLLAVGEKVYKSTNMINAIFAGKQPAATWLTLDEAREE
LEKGAAEWKWASNAKNNDEVQVVLAGIGDVPQQELMAAADKLNKLGVKFKVVNIVDLLKLQS
AKENNEALTDEEFTELFTADKPVLLAYHSYAHDVRGLIFDRPNHDNFNVHGYKEQGSTTTPY
DMVRVNDMDRYELTAEALRMVDADKYADEIKKLEDFRLEAFQFAVDKGYDHPDYTDWVWPGV
KTDKPGAVTATAATAGDNEAGGGGTSMDLFESLAQKITGKDQTIVFPEGTEPRIVGAAARLA
ADGLVKPIVLGATDKVQAVANDLNADLTGVQVLDPATYPAEDKQAMLDALVERRKGKNTPEQ
AAKMLEDENYFGTMLVYMGKADGMVSGAIHPTGDTVRPALQIIKTKPGSHRISGAFIMQKGE
ERYVFADCAINIDPDADTLAEIATQSAATAKVFDIDPKVAMLSFSTKGSAKGEMVTKVQEAT
AKAQAAEPELAIDGELQFDAAFVEKVGLQKAPGSKVAGHANVFVFPELQSGNIGYKIAQRFG
HFEAVGPVLQGLNKPVSDLSRGCSEEDVYKVAIITAAQGLA

As suggested, above, other PKL and PTA polypeptides are expected to work as described, including those having at least 70%, at least 80%, at least 90%, at least 95%, or more amino acid sequence identity to PKL and PTA, respectively, and structural and functional homologs. PKL and PTA are well-known enzymes and a large number have been cloned and characterized. Public databases include many PKL and PTA sequences.

In some embodiments, PKL and PTA polypeptides include substitutions that do not substantially affect the structure and/or function of the polypeptide. Exemplary substitutions are conservative mutations, as summarized in Table 1.

**Table 1. Exemplary amino acid substitutions**

| **Original Amino Acid Residue** | **Code** | **Acceptable Substitutions** |
|---|---|---|
| Alanine | A | D-Ala, Gly, β-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, β-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4-carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

Other linkers are also expected to work as described, including those having at least 70%, at least 80%, at least 90%, at least 95%, or more amino acid sequence identity, to Linker 1 and Linker 2. Since both tested linkers appeared to resulted in active enzymes, the nature of the linked is not believed to be critical. However, future experimentation is likely to identify linkers that optimize enzyme activity. Preferred linkers are peptides but larger linkers, including functional proteins can also be used as linkers. Such linkers may, for example, allow for isolation of an enzyme, provide a fluorescent tag, include an additional enzymatic activity, and the like. Ideally, the linker, PKL and PTA are a single contiguous amino acid sequence that can be made in a cell using normal translation machinery; however, the principle of linking PKL and PTA enzymes includes the possibility of a synthetic linker added to PKL and PTA polypeptides chemically.

In some embodiments, the yeast expressing the bifunctional PKL-PTA fusion polypeptide produce at least 1%, at least 2%, at least 3%, at least 4%, at least 5%, or even at least 6% more ethanol from a substrate than yeast lacking the bifunctional protein. In some embodiments, the yeast expressing the bifunctional PKL-PTA fusion polypeptide produce at least 1%, at least 2%, at least 3%, and even at least 4%, more ethanol from a substrate than yeast expressing separate PKL and PTA polypeptides. In some embodiments, the yeast expressing the bifunctional PKL-PTA fusion polypeptide produce at least 10%, at least 20%, at least 30%, at least 40%, at least 50%, or even at least 60% less acetate from a substrate than yeast expressing separate PKL and PTA polypeptides. In some embodiments, the yeast containing the bifunctional PKL-PTA fusion polypeptide produce at least 5%, at least 10%, at least 15%, or even at least 20%, less glycerol from a substrate than yeast lacking the bifunctional protein.

In some embodiments, the yeast expressing the bifunctional PKL-PTA fusion polypeptide do not additionally express separate PKL and PTA polypeptides. In other embodiments, the yeast expressing the bifunctional PKL-PTA fusion polypeptide also express separate PKL and/or PTA polypeptides.

### IV. Combination of PKL-PTA fusion polypeptide expression with other genetic modifications that benefit alcohol production

In some embodiments, in addition to expressing bifunctional PKL-PTA fusion polypeptides, the present modified yeast cells include additional modifications that affect ethanol production.

The modified cells may further include mutations that result in attenuation of the native glycerol biosynthesis pathway, which are known to increase alcohol production. Methods for attenuation of the glycerol biosynthesis pathway in yeast are known and include reduction or elimination of endogenous NAD-dependent glycerol 3-phosphate dehydrogenase (GPD) or glycerol phosphate phosphatase activity (GPP), for example by disruption of one or more of the genes *GPD*1, *GPD2, GPP1* and/or *GPP2.* See, *e.g.,* U.S. Patent Nos. 9,175,270 (Elke et al.), 8,795,998 (Pronk et al.) and 8,956,851 (Argyros et al.).

The modified yeast may further feature increased acetyl-CoA synthase (also referred to acetyl-CoA ligase) activity (EC 6.2.1.1) to scavenge (*i.e.*, capture) acetate produced by chemical or enzymatic hydrolysis of acetyl-phosphate (or present in the culture medium of the yeast for any other reason) and converts it to Ac-CoA. This avoids the undesirable effect of acetate on the growth of yeast cells and may further contribute to an improvement in alcohol yield. Increasing acetyl-CoA synthase activity may be accomplished by introducing a heterologous acetyl-CoA synthase gene into cells, increasing the expression of an endogenous acetyl-CoA synthase gene and the like. A particularly useful acetyl-CoA synthase for introduction into cells can be obtained from *Methanosaeta concilii* (UniProt/TrEMBL Accession No.: WP_013718460). Homologs of this enzymes, including enzymes having at least 85%, at least 90%, at least 92%, at least 95%, at least 97%, at least 98% and even at least 99% amino acid sequence identity to the aforementioned acetyl-CoA synthase from *Methanosaeta concilii,* are also useful in the present compositions and methods.

In some embodiments the modified cells may further include a heterologous gene encoding a protein with NAD⁺-dependent acetylating acetaldehyde dehydrogenase activity and/or a heterologous gene encoding a pyruvate-formate lyase. The introduction of such genes in combination with attenuation of the glycerol pathway is described, *e.g*., in U.S. Patent No. 8,795,998 (Pronk et al.). In some embodiments of the present compositions and methods the yeast expressly lack a heterologous gene(s) encoding an acetylating acetaldehyde dehydrogenase, a pyruvate-formate lyase or both.

In some embodiments, the present modified yeast cells may further overexpress a sugar transporter-like (STL1) polypeptide to increase the uptake of glycerol (see, *e.g.,* Ferreira *et al.*, 2005; Dušková *et al.*, 2015 and WO 2015023989 A1).

In some embodiments, the present modified yeast cells further include a butanol biosynthetic pathway. In some embodiments, the butanol biosynthetic pathway is an isobutanol biosynthetic pathway. In some embodiments, the isobutanol biosynthetic pathway comprises a polynucleotide encoding a polypeptide that catalyzes a substrate to product conversion selected from the group consisting of: (a) pyruvate to acetolactate; (b) acetolactate to 2,3-dihydroxyisovalerate; (c) 2,3-dihydroxyisovalerate to 2-ketoisovalerate; (d) 2-ketoisovalerate to isobutyraldehyde; and (e) isobutyraldehyde to isobutanol. In some embodiments, the isobutanol biosynthetic pathway comprises polynucleotides encoding polypeptides having acetolactate synthase, keto acid reductoisomerase, dihydroxy acid dehydratase, ketoisovalerate decarboxylase, and alcohol dehydrogenase activity.

In some embodiments, the modified yeast cells comprising a butanol biosynthetic pathway further comprise a modification in a polynucleotide encoding a polypeptide having pyruvate decarboxylase activity. In some embodiments, the yeast cells comprise a deletion, mutation, and/or substitution in an endogenous polynucleotide encoding a polypeptide having pyruvate decarboxylase activity. In some embodiments, the polypeptide having pyruvate decarboxylase activity is selected from the group consisting of: PDC1, PDC5, PDC6, and combinations thereof. In some embodiments, the yeast cells further comprise a deletion, mutation, and/or substitution in one or more endogenous polynucleotides encoding FRA2, ALD6, ADH1, GPD2, BDH1, and YMR226C.

### V. Combination of PKL-PTA fusion polypeptide expression with other beneficial mutations

In some embodiments, in addition to expressing bifunctional PKL-PTA fusion polypeptides, optionally in combination with other genetic modifications that benefit alcohol production, the present modified yeast cells further include any number of additional genes of interest encoding proteins of interest. Additional genes of interest may be introduced before, during, or after genetic manipulations that result in expression of the fusion polypeptides. Proteins of interest, include selectable markers, carbohydrate-processing enzymes, and other commercially-relevant polypeptides, including but not limited to an enzyme selected from the group consisting of a dehydrogenase, a transketolase, a phosphoketolase, a transladolase, an epimerase, a phytase, a xylanase, a β-glucanase, a phosphatase, a protease, an α-amylase, a β-amylase, a glucoamylase, a pullulanase, an isoamylase, a cellulase, a trehalase, a lipase, a pectinase, a polyesterase, a cutinase, an oxidase, a transferase, a reductase, a hemicellulase, a mannanase, an esterase, an isomerase, a pectinases, a lactase, a peroxidase and a laccase. Proteins of interest may be secreted, glycosylated, and otherwise-modified.

### VI. Use of the modified yeast for increased alcohol production

The present compositions and methods include methods for increasing alcohol production using the modified yeast in fermentation reactions. Such methods are not limited to a particular fermentation process. The present engineered yeast is expected to be a "drop-in" replacement for convention yeast in any alcohol fermentation facility. While primarily intended for fuel ethanol production, the present yeast can also be used for the production of potable alcohol, including wine and beer.

### VII. Yeast cells suitable for modification

Yeasts are unicellular eukaryotic microorganisms classified as members of the fungus kingdom and include organisms from the phyla Ascomycota and Basidiomycota. Yeast that can be used for alcohol production include, but are not limited to, *Saccharomyces* spp., including *S. cerevisiae,* as well as *Kluyveromyces, Lachancea* and *Schizosaccharomyces* spp. Numerous yeast strains are commercially available, many of which have been selected or genetically engineered for desired characteristics, such as high alcohol production, rapid growth rate, and the like. Some yeasts have been genetically engineered to produce heterologous enzymes, such as glucoamylase or α-amylase.

### VIII. Substrates and products

Alcohol production from a number of carbohydrate substrates, including but not limited to corn starch, sugar cane, cassava, and molasses, is well known, as are innumerable variations and improvements to enzymatic and chemical conditions and mechanical processes. The present compositions and methods are believed to be fully compatible with such substrates and conditions.

These and other aspects and embodiments of the present strains and methods will be apparent to the skilled person in view of the present description. The following examples are intended to further illustrate, but not limit, the strains and methods.

### EXAMPLES

### Example 1

### Materials and Methods

### Liquefact preparation

Liquefact (Ground corn slurry) was prepared by adding 600 ppm of urea, 0.124 SAPU/g ds FERMGEN™ 2.5X (acid fungal protease), 0.33 GAU/g ds glucoamylase (a variant *Trichoderma* glucoamylase) and 1.46 SSCU/g ds GC626 (*Aspergillus* α-amylase), adjusted to a pH of 4.8.

### Non-liquefied corn flour substrate preparation

A non-liquefied corn flour slurry was prepared by adding demineralized water to corn flour obtained by grinding dry corn kernels, and adjusting pH to 4.8. Further, 600 ppm of urea, 0.124 SAPU/g ds FERMGEN™ 2.5X (acid fungal protease), 0.22 mg/g ds TrGA (a *Trichoderma* glucoamylase) and 0.033 mg/g ds AcAA (*Aspergillus* α-amylase) were added.

### Serum vial assays

2 mL of YPD in 24-well plates were inoculated with yeast cells and the cells allowed to grow overnight to reach an OD between 25-30. 2.5 mL liquefact was aliquoted to serum vials (Chemglass, Catalog #: CG-4904-01) and yeast was added to each vial for a final OD of about 0.4-0.6. The lids of the vials were screw on and punctured with needle (BD, Catalog #: 305111) for ventilation (to release CO₂), then incubated at 32°C with shaking (200 RPM for 65 hours).

### AnKom assays

300 µL of concentrated yeast overnight culture was added to each of a number ANKOM bottles filled with 30 g prepared liquefact for a final OD of 0.3. The bottles were then incubated at 32°C with shaking (150 RPM) for 65 hours.

### Shake flask assays

500 µL of concentrated yeast overnight culture was added to each of a number of shake flasks filled with 100 g prepared liquefact or non-liqufied substrate for a final OD of 0.1. The flasks were incubated at 32°C or 36°C with shaking (170 RPM) for 66 hours, in the case of SSF with liquefact, or 90 hours, in the case of non-liquefied corn flour.

### HPLC analysis

Samples from serum vial and AnKom experiments were collected in Eppendorf tubes by centrifugation for 12 minutes at 14,000 RPM. The supernatants were filtered with 0.2 µM PTFE filters and then used for HPLC (Agilent Technologies 1200 series) analysis with the following conditions: Bio-Rad Aminex HPX-87H columns, running temperature of 55°C. 0.6 ml/min isocratic flow 0.01 N H₂SO₄, 2.5 µL injection volume. Calibration standards were used for quantification of the of acetate, ethanol, glycerol, and glucose. Samples from shake flasks experiments were collected in Eppendorf tubes by centrifugation for 15 minutes at 14,000 RPM. The supernatants were diluted by a factor of 11 using 5 mM H₂SO₄ and incubated for 5 min at 95°C. Following cooling, samples were filtered with 0.2 µM Corning FiltrEX CLS3505 filters and then used for HPLC analysis. 10 µl was injected into an Agilent 1200 series HPLC equipped with a refractive index detector. The separation column used was a Phenomenex Rezex-RFQ Fast Acid H+ (8%) column. The mobile phase was 5 mM H₂SO₄, and the flow rate was 1.0 mL/min at 85 °C. HPLC Calibration Standard Mix from Bion Analytical was used for quantification of the of acetate, ethanol, glycerol, and glucose. Values are expressed in g/L.

### Growth determination

2 mL 96-well microtiter plates containing 500 µL of YPD were inoculated with 20 µL concentrated yeast overnight culture to final OD of 0.1. The MTPs were incubated at 32°C or 36°C with shaking (150 RPM) for 24 hours. The yeast cultures were diluted 50 times in demineralized water to a final volume of 100 µL in 500 µL volume flat bottom transparent plates (Greiner Bio-one 655101). The OD was measured at a wavelength of 660 nm.

### Samples preparation for enzymatic activity determination

For determination of enzymatic activity of glucoamylases secreted by yeast strains grown on YPD media and on liquefact at 32°C in the shake flask assay, samples of 1 mL volume were centrifuged for 10 minutes at 14,000 RPM. The supernatants were filtered with 0.2 µM Corning FILTREX™ CLS3505 filter and used for enzymatic activity assay.

### Enzymatic activity assay

To determine enzymatic activity of secreted glucoamylases, 10 µL of sample was mixed with 90 µL 8% (w/w) maltodextrin DP4-7 (Sigma) : 50 mM NaCl buffer pH 4.3 (ratio 5:4) solution and incubated for 30 min at 32°C. The released glucose was determined with use of D-Glucose Assay Kit (GOPOD Format; Megazyme) according to supplier's instruction. A *Trichoderma* glucoamylase was used as a standard. Values are expressed in ppm.

### Example 2

### Plasmid pZK41W-GLAF12 with PKL-PTA fusion gene 1

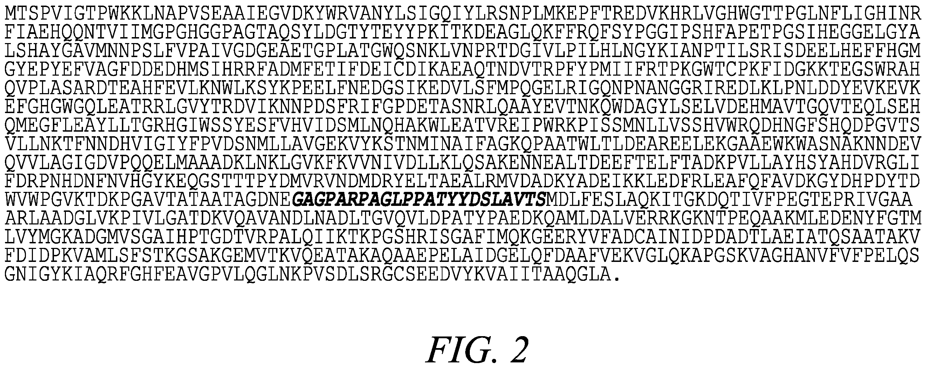

Synthetic phosphoketolase and phosphotransacetylase fusion gene 1, GvPKL-L1-LpPTA, includes the codon-optimized coding regions for the phosphoketolase from *Gardnerella vaginalis* (GvPKL; SEQ ID NO: 1) and the phosphotransacetylase from *Lactobacillus plantarum* (LpPTA; SEQ ID NO: 2) joined with synthetic linker L1 (SEQ ID NO: 3). The amino acid sequence of the resulting fusion polypeptide is shown in Figure 2 (SEQ ID NO: 5) with the linker region in bold italics:

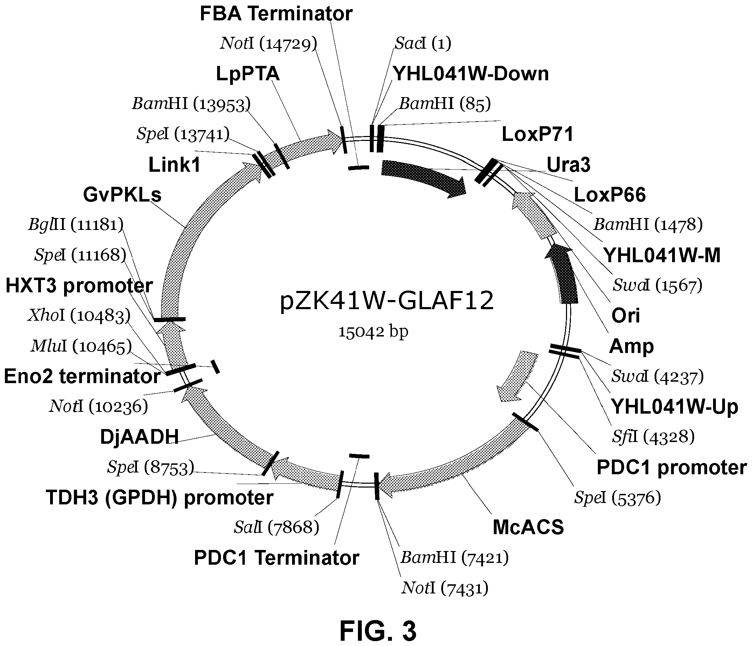

Plasmid pZK41W-GLAF12, shown in Figure 3, contains three cassettes to express the GvPKL-L1-LpPTA fusion polypeptide, acylating acetaldehyde dehydrogenase from *Desulfospira joergensenii* (DjAADH), and acetyl-CoA synthase from *Methanosaeta concilii* (McACS). Both DjAADH and McACS were codon optimized. The expression of GvPKL-L1-LpPTA is under the control of an *HXT3* promoter and *FBA1* terminator. The expression of DjAADH is under the control of *TDH3* promoter and *ENO2* terminator. The expression of McACS is under the control of *PDC1* promoter and *PDC1* terminator. Plasmid pZK41W-GLAF12 was designed to integrate the three expression cassettes into the *Saccharomyces* chromosome downstream of the *YHL041 W* locus. The functional and structural composition of plasmid pZK41W-GLAF12 is described in Table 2.

**Table 2. Functional and structural elements of plasmid pZK41W-GLAF12**

| **Location (bp)** | **Functional/Structural element** | **Description** |
|---|---|---|
| 4-81 | "Down" fragment, downstream of *YHL041W locus* | 78-bp DNA fragment (labeled as YHL041W-Down in Figure 3) from *S*. *cerevisiae* |
| 120-153 | LoxP71 site | LoxP71 site |
| 154-1442 | *Ura3* gene | *Ura3* gene used as selection marker |
| 1443-1476 | LoxP66 | LoxP66 site |
| 1483-1562 | "M" fragment, downstream of *YHL041W locus* | 80-bp DNA fragment (labeled as YHL041W-M in Figure 3) from *S. cerevisiae* |
| 1563-4242 | ColE1 replicon and ampicillin resistance marker gene | These sequences are not part of the DNA fragment integrated into yeast genome |
| 4243-4318 | "Up" fragment, downstream of *YHL041W locus* | 76-bp DNA fragment (labeled as YHL041W-Up in Fig. 3) |
| 4416-7804 | PDC1Promoter:: McACS:: PDC Terminator | Cassette for expression of codon optimized *McACS* encoding acetyl-CoA synthase, derived from *M. consilii* |
| 7873-10463 | *TDH3* Promoter:: DjAADH:: ENO Terminator | Cassette for expression of codon optimized *DjAADH* encoding acylating acetaldehyde dehydrogenase, derived from *D*. *joergensenii* |
| 10497-15037 | *HXT3 Promoter*:: GvPKL-L1-LpPTA:: FBA1 Terminator. | Cassette for expression of codon-optimized GvPKL-L1-LpPTA fusion gene encoding |

### Example 3

### Plasmid pZK41W-GLAF22 with PKL-PTA fusion gene 2

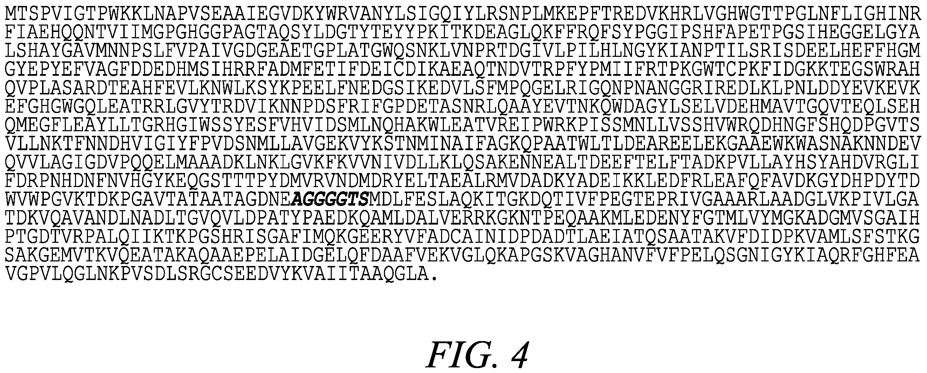

Synthetic GvPKL-L2-LpPTA includes the same phosphoketolase and phosphotransacetylase as in Example 2 joined with synthetic linker 2 (L2; SEQ ID NO 4). The amino acid sequence of the resulting fusion polypeptide is shown in Figure 4 (SEQ ID NO: 6), with the linker region in bold italics. Plasmid pZK41W-GLAF22 contains the cassette to express the GvPKL-L2-LpPTA. Plasmid pZK41W-GLAF22 (not shown) is similar to plasmid pZK41W-GLAF12 but has a different linker.

### Example 4

### Plasmid pZK41W-(H3C19) with PKL and PTA as individual genes

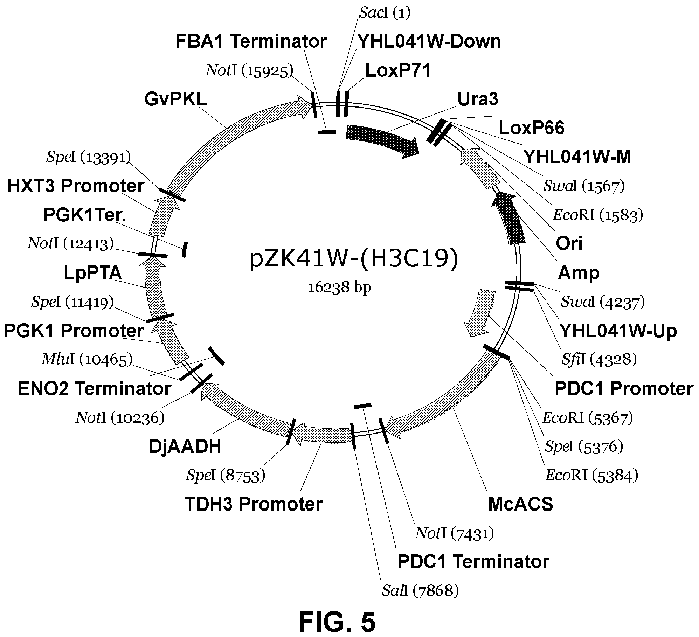

Plasmid pZK41W-(H3C19) (Figure 5) was designed as a control for testing plasmids pZK41W-GLAF12 and pZK41W-GLAF22 described, above. pZK41W-(H3C19) contains four express cassettes. The cassettes for expression of DjAADH and McACS are the same as in plasmids pZK41W-GLAF12 and pZK41W-GLAF22. However, instead of expressing GvPKL-LpPTA fusion polypeptides, the expression of the GvPKL and LpPTA are individually controlled. The expression of GvPKL is under the con trol of *HXT3* promoter and FBA1 terminator, while the expression of LpPTA is under the control of *PGK1* promoter and *PGK1* terminator. Construct pZK41W-(H3C19) is also designed to integrate the four expression cassettes into the downstream of *YHL041W* locus.

### Example 5

### Generation an FG-ura3 Strain with a ura3 genotype

The *S. cerevisiae* strain, FERMAX™ Gold Label (hereafter abbreviated, "FG") is well-known in the grain ethanol industry and was used as the parental, "wild-type" strain to make the present engineered yeast.

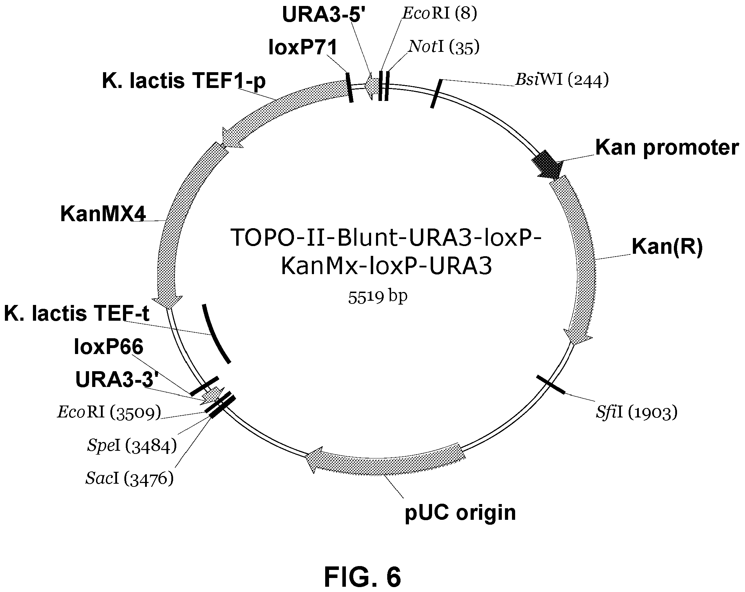

Plasmid pTOPO II-Blunt ura3-loxP-KanMX-loxP-ura3 (Figure 6) was designed to replace the *URA3* gene in strain FG with mutated ura3 and *URA3*-loxP-TEFp-KanMX-TEFt-loxP-*UR43* fragments. The functional and structural elements of the plasmid are listed in Table 3.

**Table 3. Functional/structural elements of pTOPO II-Blunt ura3-loxP-KanMX-loxP-ura3**

| **Location (bp)** | **Functional/Structural Element** | **Description** |
|---|---|---|
| 763-1695 | KanR gene in *E. coli* | Vector sequence |
| 2388-3061 | pUC origin | Vector sequence |
| 3520-3569 Reverse orientation | *URA*3 3'-flanking region, | Synthetic DNA identical to *S*. *cerevisiae* genomic sequence to *URA3* locus |
| 3601-3634 Reverse orientation | loxP66 | Synthetic DNA identical to loxP66 consensus |
| 3635-5400 Reverse orientation | TEF1::KanMX4::TEF Terminator | KanMX expression cassette |
| 5406-5439 Reverse orientation | loxP71 | Synthetic DNA identical to loxP71 consensus |
| 5470-5519 Reverse orientation | *URA3* 5'-flanking region | Synthetic DNA identical to the URA3 locus on the S. cerevisiae genome |

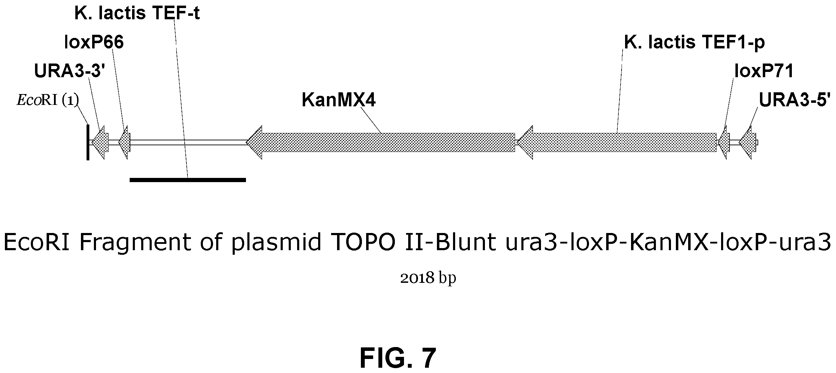

A 2,018-bp DNA fragment (Figure 7) containing the ura3-loxP-KanMX-loxP-ura3 cassette was released from plasmid TOPO II-Blunt ura3-loxP-KanMX-loxP-ura3 by *Eco*RI digestion. The fragment was used to transform *S. cerevisiae* FG cells by electroporation.

Transformed colonies able to grow on media containing G418 were streaked on synthetic minimal plates containing 20 µg/ml uracil and 2 mg/ml 5-fluoroorotic acid (5-FOA). Colonies able to grow on 5-FOA plates were further confirmed for *URA3* deletion by growth of phenotype on SD-Ura plates, and by PCR. The *ura3* deletion transformants were unable to grow on SD-Ura plates. A single 1.98-kb PCR fragment was obtained with test primers. In contrast, the same primer pairs generated a 1.3-kb fragment using DNA from the parental FG strain, indicating the presence of the intact *ura3* gene. The *ura3* deletion strain was named as FG-KanMX-ura3.

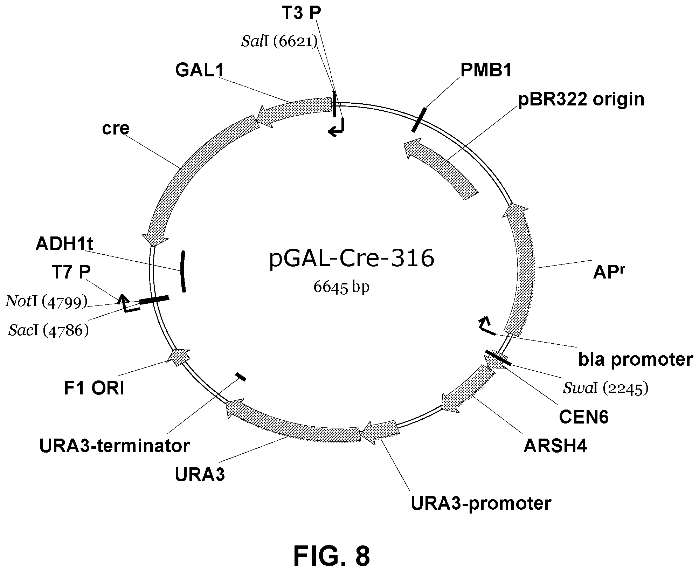

To remove the KanMX expression cassette from strain FG-KanMX-ura3, plasmid pGAL-Cre-316 (Figure 8) was used to transform cells of strain FG-KanMX-ura3 by electroporation. The purpose of using this plasmid is to temporary express the Cre enzyme, so that the LoxP-sandwiched KanMX gene will be removed from strain FG-KanMX-ura3 to generate strain FG-ura3. pGAL-Cre-316 is a self-replicating circular plasmid that was subsequently removed from strain FG-ura3. None of the sequence elements from pGAL-cre-316 was inserted into the strain FG-ura3 genome. The functional and structural elements of plasmid pGAL-Cre-316 is listed in Table 4.

**Table 4. Functional and structural elements of pGAL-Cre-316.**

| **Location (bp)** | **Functional/Structural element** |
|---|---|
| 1-4810 | Yeast-bacterial shuttle vector pRS316 sequence |
| 440-1059 | pBR322 origin of replication |
| 2984-4080 | *S. cerevisiae URA3* gene |
| 4355-4454 | F1 origin |
| 4813-6603, Reverse orientation | GALp-Cre-ADHt cassette, reverse orientation |

The transformed cells were plated on SD-Ura plates. Single colonies were transferred onto a YPG plate and incubated for 2 to 3 days at 30°C. Colonies were then transferred to new a YPD plate for 2 additional days. Finally, cell suspensions from the YPD plate were spotted on to following plates: YPD, G418 (150 µg/ml), 5-FOA (2 mg/ml) and SD-Ura. Cells able to grow on YPD and 5-FOA, and unable to grow on G418 and SD-Ura plates, were picked for PCR confirmation as described, above. The expected PCR product size was 0.4-kb and confirmed the identity of the KanMX (geneticin)-sensitive, *ura3*-deletion strain, derived from FG-KanMX-ura3. This strain was named as FG-ura3.

### Example 7

### Generation of strains expressing PKL and PTA as a fusion polypeptide or as separate polypeptides

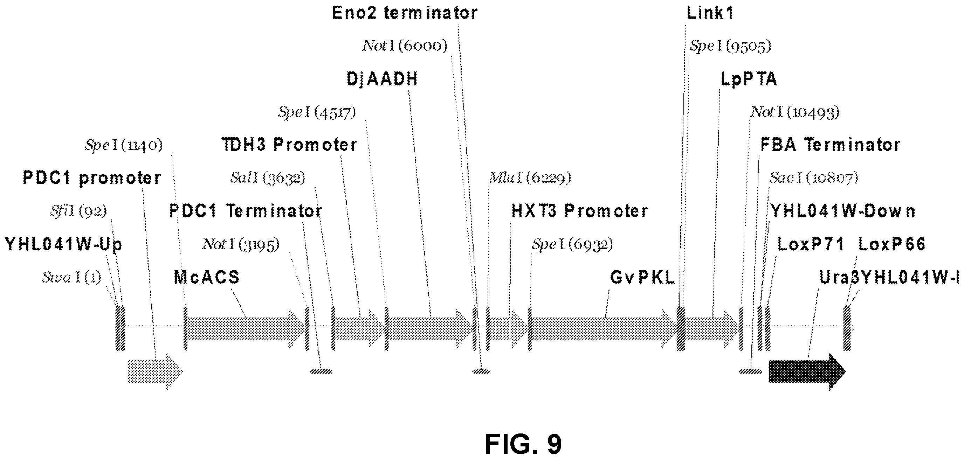

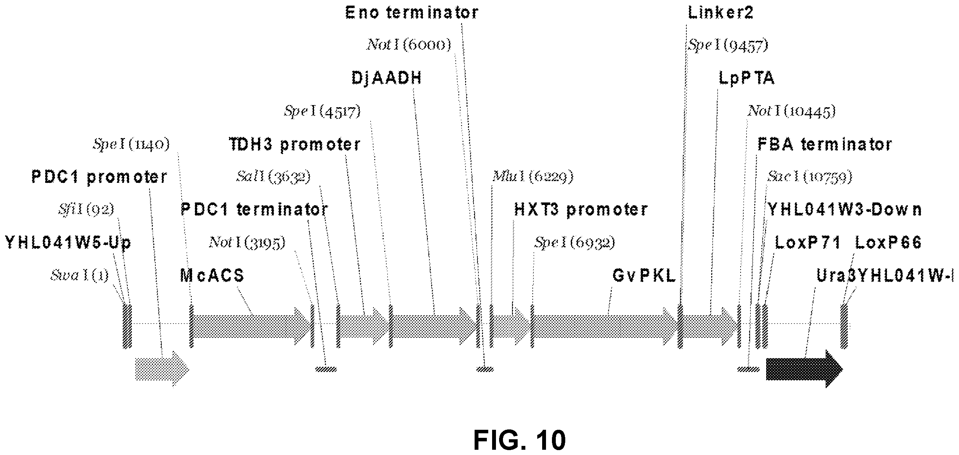

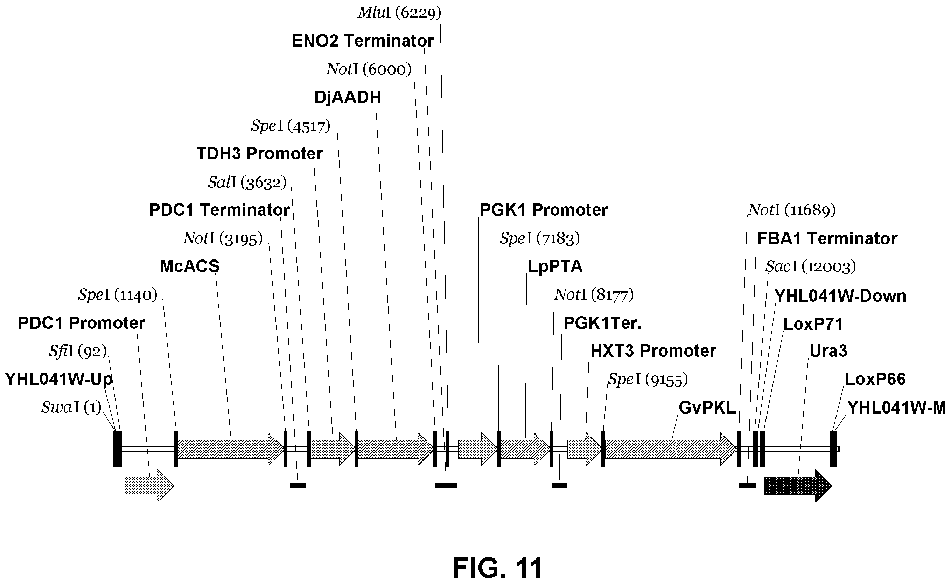

The FG-ura3 strain was used as a parent to introduce the PKL and PTA genes described, above. Cells were transformed with either (i) a 12,372-bp *Swa*I fragment containing the GvPKL-L1-LpPTA expression cassette from plasmid pZK41W-GLAF12 (Figure 9), (ii) a 12,324-bp *Swa*I fragment containing GvPKL-L2-LpPTA expression cassette from plasmid pZK41W-GLAF22 (Figure 10), or (iii) a 13,568-bp *Swa*I fragment containing GvPKL and LpPTA individual expression cassettes from plasmid pZK41W-(H3C19) (Figure 11). Transformants were selected and designated as shown in Table 5:

**Table 5. Designation of selected transformants**

| **Strain** | **Insert** | **Integration locus** | **Transgene(s) expressed** |
|---|---|---|---|
| G177 | *Swa*I fragment from pZK41W-GLAF12 (Figure 9) | *YHL041W* | GvPKL-L1-LpPTA fusion |
| G320 | *Swa*I fragment from pZK41W-GLAF22 (Figure 10) | *YHL041W* | GvPKL-L2-LpPTA fusion |
| G376 | *Swa*I fragment from pZK41W-(H3C19) (Figure 11) | *YHL041W* | GvPKL and LpPTA, individually |

### Example 8

### Comparison of strains expressing PKL and PTA as a fusion polypeptide or as separate polypeptides in vial assays

The new FG yeast strains G177, G320 and G376, along with the parent strain, FG, were grown in vial cultures and their fermentation products analyzed as described in Example 1. Performance in terms of ethanol, glycerol and acetate production (in g/L) is shown in Table 6.

**Table 6. FG versus G177, G320 and G376 in vial assays**

| **Strain** | **Transgene(s) expressed** | **EtOH** | **Glycerol** | **Acetate** |
|---|---|---|---|---|
| FG | none | 131.89 | 16.30 | 0.60 |
| G177 | GvPKL-L1-LpPTA fusion | 139.61 | 13.46 | 1.08 |
| | | | | |

| **Strain** | **Transgene(s) expressed** | **EtOH** | **Glycerol** | **Acetate** |
|---|---|---|---|---|
| FG | none | 141.58 | 18.00 | 1.04 |
| G320 | GvPKL-L2-LpPTA fusion | 146.81 | 15.83 | 1.55 |
| | | | | |

| **Strain** | **Transgene(s) expressed** | **EtOH** | **Glycerol** | **Acetate** |
|---|---|---|---|---|
| FG | none | 138.93 | 17.42 | 0.63 |
| G376 | GvPKL and LpPTA, individually | 141.28 | 14.00 | 1.43 |

All engineered yeast produced more ethanol and less glycerol than the FG parent, which is desirable in terms of performance. All the engineered yeast produced more acetate than the FG parent, which is not desirable. However, comparing the performance of strains G177, G320, G376 reveals that the increase in ethanol production is much greater with the G177 and G320 strains (5.9 and 3.7%, respectively) expressing the fusion proteins compared to the G376 strain expressing separate proteins (1.7%). In addition, the increase in acetate production is much less in the G177 and G320 strains (80 and 49%, respectively) compared to the G376 strain (127%). These results suggest that the PKL-PTA fusion polypeptide channels more sugar into ethanol and less into acetate than the separately-expressed PKL and PTA polypeptide.

### Example 9

### Comparison of strains expressing PKL and PTA as a fusion polypeptide or as separate polypeptides in AnKom assays

To confirm the benefits of the bifunctional proteins, the performance of strains G177, G320 and G376 were more precisely analyzed in AnKom assays, as described in Example 1. Performance in terms of ethanol, glycerol and acetate production (in g/L) is shown in Table 7.

**Table 7. FG versus G177, G320 and G376 in AnKom assays**

| **Strain** | **Transgene(s) expressed** | **EtOH** | **Glycerol** | **Acetate** |
|---|---|---|---|---|
| FG | none | 134.26 | 17.24 | 0.78 |
| G177 | GvPKL-L1-LpPTA fusion | 142.38 | 14.50 | 1.12 |
| G320 | GvPKL-L2-LpPTA fusion | 141.09 | 14.95 | 1.22 |
| G376 | GvPKL and LpPTA, individually | 139.39 | 13.58 | 1.54 |

The increase in ethanol production with the G177 and G320 strains was 6.0 and 5.1%, respectively, compared to only 3.9% with the G376 strain. The increase in acetate production was 44 and 56%, respectively, with the G177 and G320 strains, compared to 97% with the G376 strain.

### Example 10

### Generation of strains expressing GA together with PKL and PTA as a fusion polypeptide or as separate polypeptides

Yeast strains that express either of two different glucoamylases in combination with the fused or non-fused PKL and PTA genes, were constructed by transforming various PKL and PTA fusion or non-fusion-expressing strains with a DNA cassette containing a codon-optimized GA gene fused to the yeast FBA1 promoter and FBA1 terminator. Transformants in which the GA expression cassettes were integrated into the chromosome were identified and designated as indicated in Table 8.

**Table 8. Designation of selected GA transformants**

| **Strain** | **PKL & PTA transgene(s) expressed** | **GA expressed** |
|---|---|---|
| POL-SC-00692 | GvPKL and LpPTA, individually | GA #1 |
| POL-SC-00675 | GvPKL-L1-LpPTA fusion | GA #1 |
| POL-SC-00696 | GvPKL and LpPTA, individually | GA #2 |
| POL-SC-00699 | GvPKL-L1-LpPTA fusion | GA #2 |

### Example 11

### Comparison of the growth of strains expressing GA together with PKL and PTA as a fusion polypeptide or as separate polypeptides

To confirm the positive impact of the fusion of PKL and PTA on growth of the engineered yeast strains, the growth of pairs of otherwise isogenic strains expressing PKL and PTA either as a fusion polypeptide or as separate polypeptides were compared. The OD₆₆₀ after 24 h growth in YPD at 32°C or 36°C is shown in Table 9.

**Table 9. Comparison of the growth of strains GPY10008, GPY10009, POL-SC-00675, POL-SC-0692, POL-SC-0696 and POL-SC-00699**

| **Strain Pair** | **Strain name** | **PKL and PTA transgene(s) expressed** | **GA expressed** | **OD at 32°C** | **OD at 36°C** |
|---|---|---|---|---|---|
| 1 | POL-SC-00692 | GvPKL and LpPTA, individually | GA #1 | 3.04 | 2.48 |
| | POL-SC-00675 | GvPKL-L1-LpPTA fusion | GA #1 | 2.69 | 2.75 |
| 2 | POL-SC-00696 | GvPKL and LpPTA, individually | GA #2 | 3.05 | 2.50 |
| | POL-SC-00699 | GvPKL-L1-LpPTA fusion | GA #2 | 3.02 | 2.79 |
| 3 | GPY10008 | GvPKL and LpPTA, individually | None | 2.74 | 2.61 |
| | GPY10009 | GvPKL-L1-LpPTA fusion | None | 3.13 | 2.90 |

Comparison of the optical densities after 24 h reveals that strains GPY10009, POL-SC-00675 and POL-SC-0699, expressing the PKL-PTA fusion protein, grow better than the corresponding isogenic strains expressing PKL and PTA individually (strains GPY10008, POL-SC-00692, POL-SC-00696, respectively).

### Example 12

### Comparison of GA secretion by strains expressing PKL and PTA as a fusion polypeptide or as separate polypeptides in YPD liquid cultures

To confirm the positive influence of the expression of PKL and PTA as a single polypeptide on GA expression, enzymatic activities of the glucoamylases secreted by pairs of strains expressing PKL and PTA either as a fusion polypeptide or as separate polypeptides were compared. Table 10 shows the enzymatic activities (expressed in ppm GA) measured in the supernatants of cultures after growth on YPD at 32°C or 36°C.

**Table 10. Comparison of enzymatic activities of glucoamylases secreted by strains POL-SC-00675, POL-SC-00692, POL-SC-00696 and POL-SC-00699.**

| **Strain pair** | **Strain name** | **PKL and PTA transgene(s) expressed** | **GA expressed** | **Enzymatic activity at 32°C** | **Enzymatic activity at 36°C** |
|---|---|---|---|---|---|
| 1 | POL-SC-00692 | GvPKL and LpPTA, individually | GA #1 | 1.04 | 1.21 |
| | POL-SC-00675 | GvPKL-L1-LpPTA fusion | GA #1 | 1.11 | 1.49 |
| 2 | POL-SC-00696 | GvPKL and LpPTA, individually | GA #2 | 0.58 | 0.36 |
| | POL-SC-00699 | GvPKL-L1-LpPTA fusion | GA #2 | 0.69 | 0.63 |

Higher enzymatic activities were measured in the supernatants of strains POL-SC-00675 and POL-SC-00699 expressing the PKL-PTA fusion protein than in the supernatants from the corresponding isogenic strains expressing PKL and PTA individually (POL-SC-00692 and POL-SC-00696, respectively). This indicates higher levels of expressed glucoamylases by strains expressing fusion protein.

### Example 13

### Comparison of strains expressing GA together with PKL and PTA as a fusion polypeptide or as separate polypeptides in shake flask assay with liquefact

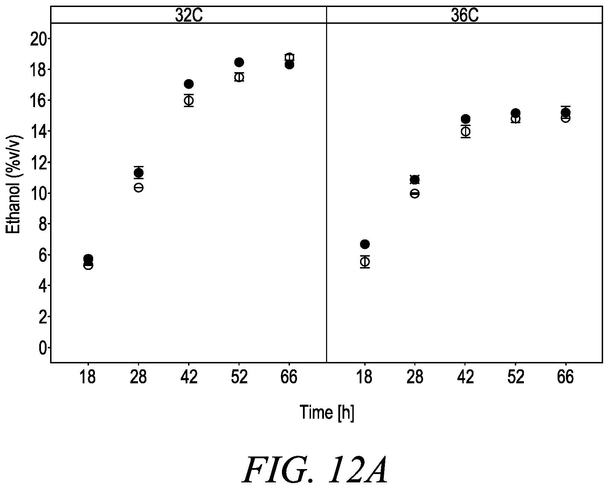

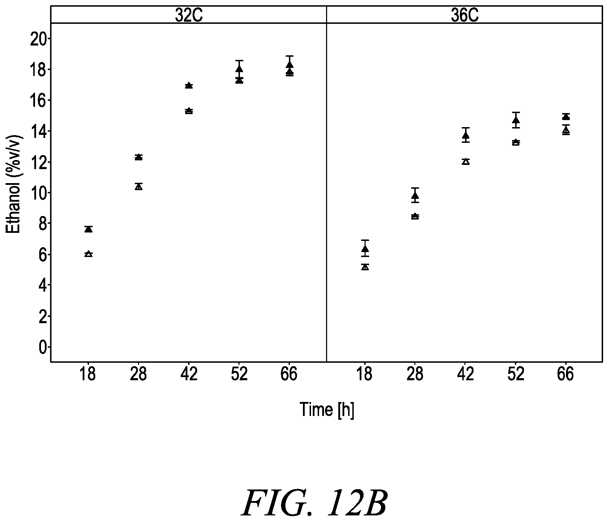

The performance in shake flasks fermentations with liquefact as a substrate of the engineered yeast strains POL-SC-00675, POL-SC-00692, POL-SC-00696 and POL-SC-00699 expressing PTA, PKL and GA was compared in order to evaluate the effect of fusion of PKL and PTA on the expression of GA during SSF process. To make the impact more visible, GA was not added to the liquefact. The obtained results are shown in Figure 12A and 12B.

Data in Figure 12 shows that strains POL-SC-00675 and POL-SC-00699 expressing the PKL-PTA fusion protein show better fermentation kinetics in comparison to the corresponding isogenic strains expressing PKL and PTA individually (POL-SC-00692 and POL-SC-00696, respectively). This indicates that the higher levels of glucoamylases being expressed by the strains expressing fused proteins, has a positive impact on the SSF process.

### Example 14

### Comparison of strains expressing GA together with PKL and PTA as a fusion polypeptide or as separate polypeptides using a non-liquefied corn flour substrate

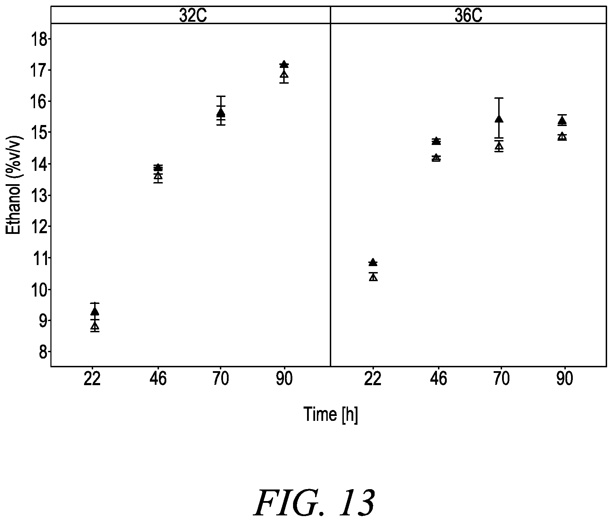

The beneficial impact of the fusion of PKL and PTA on the fermentation process was also tested in shake flask assay with non-liquefied corn flour substrate, which requires a higher dose of glucoamylases than is used for conventional liquefact. Accordingly, exogenous *Trichoderma* GA was added to the substrate to simulate the typical conditions found in an ethanol plant. The obtained results are shown in Figure 13.

Strain POL-SC-00699, expressing the PKL-PTA fusion protein, shows better fermentation kinetics and higher end-of-fermentation levels of ethanol in comparison to the corresponding isogenic strain POL-SC-00696 expressing PKL and PTA individually. This indicates higher levels of expression of glucoamylases by the strains with the PKL-PTA fusion, as well as a positive influence of the fusion of the PKL and PTA on the performance of those strains in SSF.

### Example 15

### Comparison of GA secretion by strains expressing PKL and PTA as a fusion polypeptide or as separate polypeptides using a starch liquefact substrate

To confirm that improved performance of strains expressing bifunctional protein in shake flasks assay is related to higher GA expression, enzymatic activity measurements were performed on broth obtained from fermentation cultures of strains POL-SC-00696 and POL-SC-00699. The measured enzymatic activities are presented in Table 11.

**Table 11. Comparison of the enzymatic activities in ppm GA secreted by strains POL-SC-00696 and POL-SC-00699 grown in shake flask using liquefact at 32°C.**

| **Strain** | **PKL and PTA transgene(s) expressed** | **GA expressed** | **Enzymatic activity** |
|---|---|---|---|
| POL-SC-00696 | GvPKL and LpPTA, individually | GA #1 | 1.529 |
| POL-SC-00699 | GvPKL-L1-LpPTA fusion | GA #1 | 1.572 |

Higher enzymatic glucoamylase activities were measured in the fermentation broth of strain POL-SC-00699 expressing the PKL-PTA fusion protein comparing to the corresponding isogenic strain POL-SC-00696 expressing PKL and PTA individually. This confirms that strains expressing PKL and PTA as a single polypeptide express more glucoamylase than strains in which PKL and PTA are expressed as separate proteins.

## Claims

1. A recombinant fusion polypeptide comprising a first amino acid sequence having phosphoketolase activity fused to a second amino acid sequence having phosphotransacetylase activity, wherein the fusion polypeptide, when expressed in a cell, is capable of converting fructose-6-P (F-6-P) and/or xylulose-5-P (X-5-P) to acetyl-CoA.

2. The fusion polypeptide of claim 1, wherein the first amino acid sequence and second amino acid sequence are fused via a linker peptide.

3. The fusion polypeptide of claim 1 or 2, wherein the first amino acid sequence is present at the N-terminus of the fusion polypeptide and second amino acid sequence is present at the C-terminus of the fusion polypeptide.

4. The fusion polypeptide of claim 1 or 2, wherein the second amino acid sequence is present at the N-terminus of the fusion polypeptide and first amino acid sequence is present at the C-terminus of the fusion polypeptide.

5. The fusion polypeptide of any of the preceding claims, wherein the first amino acid sequence is the phosphoketolase from *Gardnerella vaginalis.*

6. The fusion polypeptide of any of the preceding claims, wherein the second amino acid sequence is the phosphotransacetylase from *Lactobacillus plantarum.*

7. A plasmid comprising a DNA sequence encoding the fusion polypeptide of any of the preceding claims.

8. Yeast cells comprising a DNA sequence encoding the fusion polypeptide of any of claims 1-7.

9. Yeast cells expressing the fusion polypeptide of any of claims 1-7, optionally wherein the yeast cells do not additionally express separate polypeptides having phosphoketolase and/or phosphotransacetylase activity.

10. A method for increasing alcohol production or reducing acetate during fermentation, comprising contacting a carbohydrate substrate with modified yeast cells expressing the recombinant fusion polypeptide of claim 1; and allowing the yeast to produce more alcohol from the carbohydrate substrate; wherein the yeast produced less acetate that comparable yeast cells expressing the same first polypeptides and same second polypeptides as separate molecules.

11. A method for reducing acetate production by yeast cells having an exogenous phosphoketolase pathway, comprising producing in modified yeast cells the recombinant fusion polypeptide of claim 1, optionally wherein the modified yeast cells produced less acetate that comparable yeast expressing the same first polypeptides and same second polypeptides as separate molecules.

12. The method of claim 10 or 11, wherein the modified yeast cells produced at least 10% less acetate than comparable yeast expressing the same first polypeptides and same second polypeptides as separate molecules.

13. A method for increasing glucoamylase expression in yeast comprising a heterologous gene encoding a glucoamylase, comprising additionally expressing in the yeast the recombinant fusion polypeptide of claim 1; wherein the yeast produce during fermentation an increased amount of glucoamylase compared to otherwise identical yeast that produce separate molecules having phosphoketolase and/or phosphotransacetylase activity.

14. The method of any of claims 10-13, wherein the modified yeast cells do not produce separate molecules having phosphoketolase and/or phosphotransacetylase activity.

15. The method of any of claims 10-14, wherein the modified yeast is the yeast of claim 8 or 9.

## Patentansprüche

1. Rekombinantes Fusionspolypeptid, das eine erste Aminosäuresequenz mit Phosphoketolase-Aktivität, die an eine zweite Aminosäuresequenz mit Phosphotransacetylase-Aktivität fusioniert ist, umfasst, wobei das Fusionspolypeptid bei seiner Expression in einer Zelle in der Lage ist, Fructose-6-P (F-6-P) und/oder Xylulose-5-P (X-5-P) in Acetyl-CoA umzuwandeln.

2. Fusionspolypeptid nach Anspruch 1, wobei die erste Aminosäuresequenz und die zweite Aminosäuresequenz über ein Linkerpeptid fusioniert sind.

3. Fusionspolypeptid nach Anspruch 1 oder 2, wobei die erste Aminosäuresequenz an dem N-Terminus des Fusionspolypeptids vorhanden ist und die zweite Aminosäuresequenz an dem C-Terminus des Fusionspolypeptids vorhanden ist.

4. Fusionspolypeptid nach Anspruch 1 oder 2, wobei die zweite Aminosäuresequenz an dem N-Terminus des Fusionspolypeptids vorhanden ist und die erste Aminosäuresequenz an dem C-Terminus des Fusionspolypeptids vorhanden ist.

5. Fusionspolypeptid nach einem der vorangegangenen Ansprüche, wobei die erste Aminosäuresequenz die Phosphoketolase von Gardnerella vaginalis ist.

6. Fusionspolypeptid nach einem der vorangegangenen Ansprüche, wobei die zweite Aminosäuresequenz die Phosphotransacetylase von Lactobacillus plantarum ist.

7. Plasmid, das eine DNA-Sequenz umfasst, die für ein Fusionspolypeptid nach einem der vorangegangenen Ansprüche kodiert.

8. Hefezellen, die eine DNA-Sequenz umfassen, die für ein Fusionspolypeptid nach einem der Ansprüche 1 bis 7 kodiert.

9. Hefezellen, die ein Fusionspolypeptid nach einem der Ansprüche 1 bis 7 exprimieren, wobei die Hefezellen gegebenenfalls nicht zusätzlich separate Polypeptide mit Phosphoketolase- und/oder Phosphotransacetylase-Aktivität exprimieren.

10. Verfahren zur Erhöhung der Alkoholproduktion oder zur Verringerung von Acetat während der Fermentation, das das Inkontaktbringen eines Kohlenhydratsubstrats mit modifizierten Hefezellen, die ein rekombinantes Fusionspolypeptid nach Anspruch 1 exprimieren; und das Zulassen, dass die Hefe mehr Alkohol aus dem Kohlenhydratsubstrat produziert, umfasst; wobei die Hefe weniger Acetat als vergleichbare Hefezellen produziert, die die gleichen ersten Polypeptide und die gleichen zweiten Polypeptide als separate Moleküle exprimieren.

11. Verfahren zur Verringerung der Acetatproduktion von Hefezellen mit einem exogenen Phosphoketolase-Weg, das die Produktion eines rekombinanten Fusionpolypeptids nach Anspruch 1 in modifizierten Hefezellen umfasst, wobei die modifizierten Hefezellen gegebenenfalls weniger Acetat als vergleichbare Hefe produzieren, die die gleichen ersten Polypeptide und die gleichen zweiten Polypeptide als separate Moleküle exprimiert.

12. Verfahren nach Anspruch 10 oder 11, wobei die modifizierten Hefezellen zumindest 10 % weniger Acetat als vergleichbare Hefe produzieren, die die gleichen ersten Polypeptide und die gleichen zweiten Polypeptide als separate Moleküle exprimiert.

13. Verfahren zur Erhöhung der Glucoamylase-Expression in Hefe, die ein heterologes Gen umfasst, das für eine Glucoamylase kodiert, das das zusätzliche Exprimieren eines rekombinanten Fusionpolypeptids nach Anspruch 1 in der Hefe umfasst; wobei die Hefe während der Fermentation eine erhöhte Menge von Glucoamylase produziert, verglichen mit einer ansonsten identischen Hefe, die separate Moleküle mit Phosphoketolase- und/oder Phosphotransacetylase-Aktivität produziert.

14. Verfahren nach einem der Ansprüche 10 bis 13, wobei die modifizierten Hefezellen keine separaten Moleküle mit Phosphoketolase- und/oder Phosphotransacetylase-Aktivität produzieren.

15. Verfahren nach einem der Ansprüche 10 bis 14, wobei die modifizierte Hefe eine Hefe nach Anspruch 8 oder 9 ist.

## Revendications

1. Polypeptide de fusion recombinant comprenant une première séquence d'acides aminés ayant une activité phosphocétolase fusionnée à une seconde séquence d'acides aminés ayant une activité phosphotransacétylase, dans lequel le polypeptide de fusion, lorsqu'il est exprimé dans une cellule, est capable de convertir du fructose-6-P (F-6-P) et/ou du xylulose-5-P (X-5-P) en acétyl-CoA.

2. Polypeptide de fusion selon la revendication 1, dans lequel la première séquence d'acides aminés et la seconde séquence d'acides aminés sont fusionnées via un peptide de liaison.

3. Polypeptide de fusion selon la revendication 1 ou 2, dans lequel la première séquence d'acides aminés est présente à l'extrémité N-terminale du polypeptide de fusion et la seconde séquence d'acides aminés est présente à l'extrémité C-terminale du polypeptide de fusion.

4. Polypeptide de fusion selon la revendication 1 ou 2, dans lequel la seconde séquence d'acides aminés est présente à l'extrémité N-terminale du polypeptide de fusion et la première séquence d'acides aminés est présente à l'extrémité C-terminale du polypeptide de fusion.

5. Polypeptide de fusion selon l'une quelconque des revendications précédentes, dans lequel la première séquence d'acides aminés est la phosphocétolase provenant de *Gardnerella vaginalis.*

6. Polypeptide de fusion selon l'une quelconque des revendications précédentes, dans lequel la seconde séquence d'acides aminés est la phosphotransacétylase de *Lactobacillus plantarum.*

7. Plasmide comprenant une séquence d'ADN codant pour le polypeptide de fusion selon l'une quelconque des revendications précédentes.

8. Cellules de levure comprenant une séquence d'ADN codant pour le polypeptide de fusion selon l'une quelconque des revendications 1 à 7.

9. Cellules de levure exprimant le polypeptide de fusion selon l'une quelconque des revendications 1 à 7, facultativement dans lesquelles les cellules de levure n'expriment pas en plus des polypeptides séparés ayant une activité phosphocétolase et/ou phosphotransacétylase.

10. Procédé pour augmenter la production d'alcool ou réduire de l'acétate pendant une fermentation, comprenant les étapes consistant à mettre en contact un substrat d'hydrate de carbone avec des cellules de levure modifiée exprimant le polypeptide de fusion recombinant selon la revendication 1 ; et permettre à la levure de produire plus d'alcool à partir du substrat d'hydrate de carbone ; dans lequel la levure produit moins d'acétate que des cellules de levure comparables exprimant les mêmes premiers polypeptides et les mêmes seconds polypeptides sous la forme de molécules séparées.

11. Procédé pour réduire une production d'acétate par des cellules de levure ayant une voie de phosphocétolase exogène, comprenant la production dans des cellules de levure modifiée du polypeptide de fusion recombinant de la revendication 1, facultativement dans lequel les cellules de levure modifiée ont produit moins d'acétate que de la levure comparable exprimant les mêmes premiers polypeptides et les mêmes deuxièmes polypeptides sous la forme de molécules séparées.

12. Procédé selon la revendication 10 ou 11, dans lequel les cellules de levure modifiée ont produit au moins 10 % en moins d'acétate que la levure comparable exprimant les mêmes premiers polypeptides et les mêmes seconds polypeptides sous la forme de molécules séparées.

13. Procédé pour augmenter une expression de glucoamylase dans la levure comprenant un gène hétérologue codant pour une glucoamylase, comprenant en outre une expression dans la levure du polypeptide de fusion recombinant selon la revendication 1 ; dans lequel la levure produit pendant une fermentation une quantité accrue de glucoamylase par comparaison à une levure par ailleurs identique qui produit des molécules séparées ayant une activité phosphocétolase et/ou phosphotransacétylase.

14. Procédé selon l'une quelconque des revendications 10 à 13, dans lequel les cellules de levure modifiée ne produisent pas de molécules séparées ayant une activité phosphocétolase et/ou phosphotransacétylase.

15. Procédé selon l'une quelconque des revendications 10 à 14, dans lequel la levure modifiée est la levure selon la revendication 8 ou 9.
